(19) Europäisches Patentamt
European Patent Office
Office européen des brevets

(11) EP 2 819 643 B1

(12) **EUROPEAN PATENT SPECIFICATION**

(45) Date of publication and mention of the grant of the patent:
**18.10.2017 Bulletin 2017/42**

(21) Application number: **13708638.5**

(22) Date of filing: **01.03.2013**

(51) Int Cl.:
***A61K 8/894*** *(2006.01)* ***A61K 8/37*** *(2006.01)*
***A61Q 17/04*** *(2006.01)* ***A61K 8/06*** *(2006.01)*

(86) International application number:
**PCT/US2013/028578**

(87) International publication number:
**WO 2013/130948 (06.09.2013 Gazette 2013/36)**

(54) **SUNSCREEN COMPOSITION COMPRISING UV COMPOSITE**

SONNENSCHUTZZUSAMMENSETZUNG MIT UV-VERBUNDSTOFF

COMPOSITION DE PROTECTION SOLAIRE COMPRENANT UN COMPOSITE UV

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2012 US 201261605535 P**

(43) Date of publication of application:
**07.01.2015 Bulletin 2015/02**

(73) Proprietor: **The Procter & Gamble Company**
**Cincinnati, OH 45202 (US)**

(72) Inventor: **TANNER, Paul, Robert**
**Cincinnati, Ohio 45201 (US)**

(74) Representative: **Engisch, Gautier**
**NV Procter & Gamble Services Company SA**
**IP Patent Department**
**Temselaan 100**
**1853 Strombeek-Bever (BE)**

(56) References cited:
**EP-A1- 1 426 027**
**WO-A2-2007/130412**
**US-A1- 2004 228 821**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 819 643 B1

## Description

### FIELD OF THE INVENTION

As are herein described comprising a UV composite comprising a silicone elastomer swollen in a liquid UV active.

### BACKGROUND OF THE INVENTION

When applied topically, sunscreen compositions impart a film that protects skin against the damaging effects of exposure to the sun's ultraviolet radiation (UV). Sunscreen compositions comprise UV actives that absorb UV before it can interact with and damage skin. A particular composition's ability to block UV is usually expressed in a sun protection factor (SPF) rating. Combinations of UV active compounds are typically used in sunscreen composition in order to raise the SPF rating of the composition and to offer broad spectrum protection. A variety of products providing protection to the skin and other keratinous tissue from the harmful effects of ultraviolet radiation (UV) are commercially available. Creating sunscreen compositions typically involves balancing of factors. Formulators wish to maximize the efficacy of the composition while minimizing trade-offs. While the efficacy of sunscreen compositions can be improved by increasing the amount of UV actives present, highly effective UV actives have a consumer disfavored heavy, oily skin feel. Increasing the amount of such UV actives can result in products that are undesirable to the consumer.

Silicone elastomers may be used to counteract the poor aesthetics, but silicone elastomers add further challenges to the formulation and are typically quite expensive. Silicone elastomers are well known for providing a unique, consumer desirable feel profile. Silicone elastomers can impact UV active solubility (which directly impacts efficacy of UV protection if the sunscreen should precipitate) and overall product stability. A problem with conventional silicone elastomer is that silicone elastomers are either synthesized and/or swollen in solvents that may be incompatible with UV actives. In such cases, a formulators ability load UV actives into the composition may be limited or additional excipients may be required to make the UV actives more compatible. Even if the solvent and sunscreen agent do not pose a compatibility issue, the solvent is often a material that provides little to no efficacy for keratinous tissue. Furthermore, the solvent adds to the heavy, oily feel already associated with UV actives.

### SUMMARY OF THE INVENTION

A sunscreen composition in the form of an emulsion may comprise from 2% to 40% of a UV composite comprising from 7% to 50%, by weight of the UV composites, of a silicone elastomer swollen in a liquid UV active such that the ratio of liquid UV active to silicone elastomer is from 1:1 to 8:1. The sunscreen composition may also comprise from 3% to 50% of an oil phase, wherein the liquid UV active comprises at least 60% by weight of the oil phase; and from 40 to 90% of an aqueous phase.

A SPF enhancing UV composite for addition to a sunscreen composition is also described herein. The UV SPF enhancing composite comprises a silicone elastomer swollen in a liquid UV active such that the ratio of liquid UV active to silicone elastomer is from 1:1 to 8:1.

A method is described for boosting the SPF of a sunscreen composition comprising the step of preparing a UV composite by swelling a silicone elastomer in a liquid organic UV active such that the ratio of liquid UV active to silicone elastomer is from 1:1 to 8:1; preparing a carrier system comprising at least an aqueous phase; and dispersing the UV composite in the carrier system.

A sunscreen composition is also described in the form of an emulsion comprising from 5% to 60% of an oil phase, wherein the oil phase comprising at least 60%, by weight of the oil phase, of a liquid UV active; and from 0.5 to 10%, by weight of the composition, of a polysiloxane elastomer; wherein the ratio of liquid organic UV active to polysiloxane elastomer is from 1:1 to 8:1.

US2004/228821 and WO 2007/130412 disclose compositions comprising UV actives.

### DETAILED DESCRIPTION OF THE INVENTION

All percentages and ratios used herein are by weight of the total composition, unless otherwise designated. All measurements are understood to be made at ambient conditions, where "ambient conditions" means conditions at about 25 °C, under about one atmosphere of pressure, and at about 50% relative humidity, unless otherwise designated. All numeric ranges are inclusive of narrower ranges; delineated upper and lower range limits are combinable to create further ranges not explicitly delineated.

The compositions of the present invention can comprise, consist essentially of, or consist of, the essential components as well as optional ingredients described herein. As used herein, "consisting essentially of" means that the composition or component may include additional ingredients, but only if the additional ingredients do not materially alter

the basic and novel characteristics of the claimed compositions or methods.

"Apply" or "application," as used in reference to a composition, means to apply or spread the compositions of the present invention onto keratinous tissue such as the epidermis.

"Keratinous tissue" refers to keratin-containing layers disposed as the outermost protective covering of mammals (*e.g.*, humans, dogs, cats, etc.) which includes, but is not limited to, skin, lips, hair, toenails, fingernails, cuticles, hooves, etc.

"Dermatologically acceptable" means that the compositions or components described are suitable for use in contact with human skin tissue without undue toxicity, incompatibility, instability, allergic response, and the like.

"Safe and effective amount" means an amount of a compound or composition sufficient to significantly induce a positive benefit.

"Non-UV" means a material not recognized by a skilled artisan in the field of sunscreen formulation to be a dermatologically acceptable UV active absorbing material.

"UV active" means a material recognized by a skilled artisan in the field of sunscreen formulation to be a dermatologically acceptable UV active absorbing material. Such UV actives may be described as being UV-A and/or UV-B active agents. Approval by a regulatory agency is generally required for inclusion of active agents in formulations intended for human use. Those active agents which have been or are currently (per 21 C.F.R. part 352) approved by the U.S. Food and Drug Administration as acceptable for use in over-the counter sunscreen drug products include organic and inorganic substances including, without limitation, para aminobenzoic acid, avobenzone, cinoxate, dioxybenzone, homosalate, menthyl anthranilate, octyl salicylate, oxybenzone, padimate O, phenylbenzimidazole sulfonic acid, sulisobenzone, trolamine salicylate, titanium dioxide, zinc oxide, diethanolamine methoxycinnamate, digalloy trioleate, ethyl dihydroxypropyl PABA, glyceryl aminobenzoate, lawsone with dihydroxyacetone, red petrolatum. Examples of other UV actives that have not yet been approved in the U.S. but are approved for over the counter use in other regions or countries such as Europe (per European Commission's Cosmetic Directive Regulation), Japan, China, Australia, New Zealand, or Canada include ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidenecamphor, and isopentyl 4-methoxycinnamate. However, as the list of approved materials is currently expanding, those of ordinary skill will recognize that the invention is not limited to UV actives currently approved for human use but includes those UV actives that may be allowed in the future.

"Leave-on," in reference to compositions, means compositions intended to be applied to and allowed to remain on the keratinous tissue. These leave-on compositions are to be distinguished from compositions which are applied to the skin and subsequently (in a few minutes or less) removed either by washing, rinsing, wiping, or the like. Leave-on compositions exclude rinse-off applications such as shampoos, facial cleansers, hand cleansers, body wash, or body cleansers. The leave-on compositions may be substantially free of cleansing or detersive surfactants. For example, "leave-on compositions" may be left on the keratinous tissue for at least 15 minutes. For example, leave-on compositions may comprise less than 1% detersive surfactants, less than 0.5% detersive surfactants, or 0% detersive surfactants. The compositions may, however, contain emulsifying or other processing surfactants that are not intended to provide any significant cleansing benefits when applied topically to the skin.

"Derivatives" means an ester, ether, amide, hydroxy, and/or salt structural analogue of the relevant compound.

"Soluble" means at least about 0.1 g of solute dissolves in 100 ml of solvent, at 25 °C and 1 atm of pressure.

The sunscreen composition may involve a wide variety of forms. Non-limiting examples include simple solutions (*e.g.*, water or oil based), dispersions, and emulsions. The sunscreen compositions may be fluid or solid so as to include lotions, creams, gels, serums, sticks, flowable solids, amorphous materials. In certain embodiments, the sunscreen composition is in the form of an emulsion. Emulsion may be generally classified as having a continuous aqueous phase (*e.g.,* oil-in-water, water-in-oil-in-water, and the like) or a continuous oil phase (*e.g.,* water-in-oil, oil-in-water-in-oil, and the like).

UV Composite

The sunscreen composition comprises a UV composite. The UV composite comprises a silicone elastomer fully or partially swollen in a liquid UV active. The UV composite may be in the form of discrete particles (*i.e.,* UV composite particles). The UV composite may be in the form of a relatively homogenous structure or gel. The UV composite is such that the liquid UV active is entrapped or dispersed within the silicone elastomer and excludes free liquid UV active that maybe decanted from the UV composite. In some embodiments, the UV composite may consist or consist essentially of the silicone elastomer being fully or partially swollen with the liquid UV active. In other embodiments, the UV composite may comprise the silicone elastomer being fully or partially swollen with the liquid UV active and a non-UV solvent.

The UV composite may be pre-formed by swelling the silicone elastomer in the liquid UV active prior to combining

other materials in the sunscreen composition. Many of the commercial silicone elastomer presented may be sold pre-swollen in a non-UV solvent. In such cases the silicone elastomer may be de-swollen to remove the non-UV solvent prior to incorporation in the sunscreen composition. A suitable method for deswelling of these commercial materials is to cast the pre-swollen silicone elastomer as a thin film. The film is allowed to dry thereby allowing vaporization of the solvent. The drying process may be accelerated through the use of heat and/or reduced pressure. The particular drying conditions will depend upon the non-UV solvent present in the pre-swollen silicone elastomer. Once dry, the silicone elastomer is recovered and re-swollen in the liquid UV active. In other embodiments, the UV composite may be formed during the polymerization of the silicone elastomer in the presence of one or more liquid UV actives which serve as the reaction solvent. In other embodiments, the UV composite may be formed within the sunscreen composition when the silicone elastomer swells with the available solvents present in the oil phase including the liquid UV active.

The composition may comprise any suitable amount of UV composite to provide the desired UV protection and/or skin feel benefit. In certain embodiments, the sunscreen composition comprises from about 2%, 5%, 10%, 15%, 20%, or 25% to about 40%, 30%, 20%, 10% or 5% of the UV composite. In a select embodiment, the sunscreen composition comprises from about 2% to about 40% of UV composite.

Silicone elastomers suitable for use in the sunscreen composition may be formed by a variety of conventional reaction pathways. Suitable reaction pathways include 1) addition reaction-curing organopolysiloxane compositions which cure under platinum metal catalysis by the addition reaction between Si-H containing organopolysiloxanes and organopolysiloxanes having silicon-bonded vinyl groups or an organic having vinyl groups; 2) condensation-curing organopolysiloxane compositions which cure in the presence of an organotin compound by a dehydrogenation reaction between hydroxyl-terminated diorganopolysiloxane and Si-H containing organopolysiloxanes; 3) condensation-curing organopolysiloxane compositions which cure in the presence of an organotin compound or a titanate ester, by a condensation reaction between a hydroxyl-terminated organopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); 4) peroxide-curing organopolysiloxane compositions which thermally cure in the presence of an organoperoxide catalyst; and 5) organopolysiloxane compositions which are cured by high-energy radiation, such as by gamma-rays, ultraviolet radiation, or electron beams. Conventional reactions are typically carried out in a solvent. Conventional solvents used in the silicone elastomer synthesis include ethanol, isododecane, and volatile silicone fluids such as dimethicone. The solvent is removed post-reaction using well known isolation techniques to recover the silicone elastomer. The UV composite may be formed by utilizing a liquid UV active (e.g., octisalate, homosalate, etc.) in lieu of or in addition to conventional solvents.

The UV composite may comprise varying amounts of silicone elastomer. The amount of silicone elastomer present in the UV composite is often dependent upon the particular silicone elastomer, liquid UV active, and, if present, non-UV solvent. In certain embodiments, the UV composite comprises the silicone elastomer in any range selected from the weight percentages, by weight of the UV composite, 7%, 9%, 10%, 11%, 14%, 15%, 16%, 20%, 25%, 33%, or 50%. In a suitable embodiment, the UV composite comprises from about 7% to about 50%, by weight of the UV composite, of the silicone elastomer. The weight percentage of silicone elastomer present in the sunscreen composition may be readily calculated by multiplying the weight percentage of the UV composite in the sunscreen composition by the weight percentage of silicone elastomer in the UV composite.

In certain embodiments, the silicone elastomer is non-spherical. The shape of the silicone elastomer may be determined microscopically. Alternately, the spherical shape can be quantified by circularity. Circularity is a quantitative, 2-dimension image analysis shape description as measured according to ISO 9276-6:2008(E) section 8.2 as implemented via the Occhio Nano 500 Particle Characterization Instrument with its accompanying software Callistro version 25 (Occhio s.a. Liege, Belgium). Circularity values range from 0 to 1, where a circularity of 1 describes a perfectly spherical particles or disc particle as measured in a two dimensional image. Circularity is calculated by the following equation:

$$C = \sqrt{\frac{4\pi A}{P^2}}$$

wherein A is projection area, which is a 2D descriptor, and P is the perimeter length of the particle. The silicone elastomers, in certain embodiments, have a circularity of less than 0.98, 0.95, or 0.9.

In select embodiments, the UV composite comprises a silicone elastomer having organic modification. Suitable silicone elastomers having organic modification include silicone elastomers having alkoxy modification. The alkoxy modification may by inclusion of polyoxyethylene, polyoxypropylene or polyglycerin units. Suitable alkoxy modified silicone elastomer may be formed by reaction pathways well known in the art. Suitable pathways are described in U.S. Patent No. 5,236,986, 5,387,417, 5,412,004, 5,811,487, and U.S. Patent Application Publication Nos. 2006/0013791A1, 2006/0034875A1, 2010/0183525A1, 2010/0172849A1, and 2010/0158824A1.

Suitable silicone elastomers include many commercial materials. An exemplary polyoxyethylene silicone elastomer includes dimethicone/PEG-10/15 crosspolymer (KSG-210 from Shin-Etsu Chemical Co, Ltd.). Examples of polyoxypropylene silicone elastomers include dimethicone/bis-isobutyl PPG-20 crosspolymer (Dow Corning EL-8050, EL-8051, & EL-8052 from Dow Corning Corp.). An exemplary polyoxyethylene/polyoxypropylene silicone elastomers is PEG-12 Dimethicone/ PPG-20 crosspolymer (available from Dow Corning)

Examples of alkyl-containing polyoxyethylene silicone elastomers include PEG-15 lauryl dimethicone crosspolymer (KSG-310, KSG-320, KSG-330, & KSG-340 from Shin-Etsu Chemical Co., Ltd.).

An example of polyglycerin-modified silicone elastomer includes dimethicone/ polyglycerin-3 crosspolymer (KSG-710 from Shin-Etsu Chemical Co., Ltd.).

Examples of alkyl-containing polyglycerin-modified silicone elastomers include lauryl dimethicone/polyglycerin-3 crosspolymer (KSG-810, KSG-820, KSG-830, & KSG-840 from Shin-Etsu Chemical Co., Ltd.).

Suitable silicone elastomers include silicone elastomers having organic modification with no alkoxy modification. Suitable organic modified silicone elastomer may be formed by reaction pathways well known in the art. Suitable pathways are described in U.S. Patent Nos. 5,654,389 and 6,262,170. Suitable silicone elastomers include many commercial materials. Examples of alkyl-containing methylpolysiloxane elastomers include C30-45 alkyl cetearyl dimethicone crosspolymer (Velvesil 125 and 034 from Momentive Performance Materials Inc.) and vinyl dimethicone/lauryl dimethicone crosspolymer (KSG-41, KSG-42, KSG-43, & KSG-44 from Shin-Etsu Chemical Co., Ltd. and Silwax CR 5012-1 from Siltech LLC).

In select embodiments, the silicone elastomer is chosen from alkyl dimethicone/polyglycerin crosspolymers, dimethicone/polyglycerin crosspolymers, dimethicone/poly(propylene glycol) crosspolymers, dimethicone/poly(ethylene glycol) crosspolymers, alkyl dimethicone/poly(propylene glycol) crosspolymers, alkyl dimethicone/poly(ethylene glycol) crosspolymers, and alkyl dimethicone crosspolymers.

Many of the commercial examples presented above have the silicone elastomer swollen in a solvent that is not a UV active. In such cases the silicone elastomer is to be deswollen to remove the non-UV solvent prior to incorporation in the sunscreen composition. A suitable method for deswelling of commercial elastomers swollen in a volatile solvent is to cast the commercial elastomer into a thin film on a inert substrate, allow the product film to dry, and then recover the remaining solvent-free elastomer. This solvent-free elastomer can then be swollen in one or more UV actives.

Alternatively, the silicone elastomer can be synthesized in the presence of one or more liquid UV actives which serve as the reaction solvent, thus avoiding the need to deswell the elastomer.

The UV composite comprises a liquid UV active. The liquid UV active may comprise one or more UV actives. Suitable UV actives that are liquid at ambient conditions include 2-ethylhexyl-p-methoxycinnamate, homomethyl salicylate, octyldimethyl-p-aminobenzoic acid, 2-ethylhexyl-2-cyano-3,3-diphenylacrylate, 2-ethylhexyl-salicylate, 3,3,5-trimethylcyclohexylsalicylate, methyl anthranilate, and mixtures of these compounds. In certain embodiments, the liquid UV active is selected from 2-ethylhexyl-p-methoxycinnamate, 2-ethylhexyl salicylate, and combinations thereof.

The UV composite may comprise varying amounts of liquid UV active. The amount of liquid UV active present in the UV composite is often dependent upon the particular silicone elastomer, liquid UV active, and, if present, non-UV solvent. In certain embodiments, the UV composite comprises the liquid UV active in a weight ratio of liquid UV active to silicone elastomer of from 1:1 to 8:1. In other embodiments, the weight ratio of liquid UV active to silicone elastomer is in any range selected from the endpoints of 1:1, 2:1, 3:1, 4:1, 5:1, 6:1, 7:1, 8:1. The aforementioned weight ratios refer to the UV composite wherein the liquid UV active is entrapped or dispersed within the silicone elastomer. These ratios do not include free liquid UV active that maybe decanted from the UV composite. It should be recognized that additional free liquid UV active may be added to the sunscreen formulation in addition to the liquid UV active within the UV composite. The sunscreen composition as a whole may comprise from about 0.1%, 0.5%, 1%, 2%, 3%, 5% to about 40%, 30%, 20%, 10%, 7%, or 5%, by weight of the composition, of UV active whether as part of the UV composite or as free liquid.

The liquid UV active may also include UV actives that are solid at ambient conditions but are dissolved in a solvent. The solvent may be a liquid UV active (e.g., octisalate) or a non-UV liquid (e.g., isopropyl lauroyl sarcosinate, C12-15 alkyl benzoates, diisopropyl adipate, etc.). If a non-UV liquid is used to solubilize the solid UV active, the weight of the UV active and not the non-UV liquid is used in calculating the weight ratio of liquid UV active to silicone elastomer. The UV actives that are solid at ambient conditions include dibenzoylmethane derivatives such as 4-tert-butyl-4'-methoxy dibenzoylmethane (*i.e.,* butyl methoxydibenzoylmethane or avobenzone)(commercially available as PARSOL® 1789 from DSM). Other suitable solid UV actives include bis-ethylhexyloxyphenol methoxyphenyl triazine (*i.e.*, bemotrizinol, commercially available as Tinosorb® S from BASF), 2,4,6-trianilino-(p-carbo-2'-ethylhexyl-1'-oxy)-1,3,5-triazine (*i.e.,* ethylhexyl triazone commercially available as Uvinul® T 150 from BASF), diethylhexyl butamido triazone (*i.e.,* iscotrizinol, commercially available as Uvasorb® HEB by 3V Sigma), diethylamino hydroxybenzoyl hexyl benzoate (commercially available as Uvinul® A Plus from BASF), benzophenone-3 (*i.e.,* (2-Hydroxy-4-methoxyphenyl)-phenylmethanone or oxybenzone, available Eusolex 4360 from EMD Chemical, Inc.), 4-methylbenzylidene camphor (commercially available as PARSOL® 5000 from DSM), ethylhexyl bis-isopentylbenzoxazolylphenyl melamine (commercially available as Uvasorb® k2A by 3V Sigma), and combinations thereof.

The UV composite may comprise other non-UV solvents. Suitable non-UV solvents include oils listed herein. In certain embodiments the UV composite consists or consists essentially of the silicone elastomer and liquid UV active; however, a skilled artisan will recognized that when the UV composite is present in the sunscreen composition some degree of liquid migration may occur. The liquid UV active may migrate from the UV composite and/or non-UV solvents or oils may migrate into the UV composite. Likewise, a residual amount of non-UV solvents may be present in the silicone elastomer even after deswelling, but such amounts are considered insignificant in comparison the UV liquid present in the UV composite.

Sunscreen Composition

The sunscreen composition may comprise an oil phase comprising materials in addition to the UV composite. The oil phase may comprise oils, emollients, and other oil-soluble materials such as oil soluble vitamins. In some embodiments, the oil phase comprises at least about 60%, by weight of the oil phase, of UV active. In other embodiments, the oil phase comprises at least about 60%, by weight of the oil phase, of liquid UV active.

The oil phase may comprise oils which may be used to solubilize, disperse, or carry materials that are not suitable for water or water soluble solvents. Suitable oils include silicones, hydrocarbons, esters, amides, ethers, and mixtures thereof.

Suitable silicone oils include polysiloxanes. Polylsiloxanes may have a viscosity of from about 0.5 to about 1,000,000 centistokes at 25°C. Such polysiloxanes can be represented by the general chemical formula:

$$R_3SiO[R_2SiO]_xSiR_3$$

wherein R is independently selected from hydrogen or $C_{1-30}$ straight or branched chain, saturated or unsaturated alkyl, phenyl or aryl, trialkylsiloxy; and x is an integer from 0 to about 10,000, chosen to achieve the desired molecular. In certain embodiments, R is hydrogen, methyl, or ethyl. Commercially available polysiloxanes include the polydimethylsiloxanes, which are also known as dimethicones, examples of which include the DM-Fluid series from Shin-Etsu, the Vicasil® series sold by Momentive Performance Materials Inc., and the Dow Corning® 200 series sold by Dow Corning Corporation. Specific examples of suitable polydimethylsiloxanes include Dow Coming® 200 fluids (also sold as Xiameter® PMX-200 Silicone Fluids) having viscosities of 0.65, 1.5, 50, 100, 350, 10,000, 12,500 100,000, and 300,000 centistokes.

Suitable dimethicones include those represented by the chemical formula:

$$R_3SiO[R_2SiO]_x[RR'SiO]_ySiR_3$$

wherein R and R' are each independently hydrogen or $C_{1-30}$ straight or branched chain, saturated or unsaturated alkyl, aryl, or trialkylsiloxy; and x and y are each integers of 1 to 1,000,000 selected to achieve the desired molecular weight. Suitable silicones include phenyl dimethicone (Botansil™ PD-151 from Botanigenics, Inc.), diphenyl dimethicone (KF-53 and KF-54 from Shin-Etsu), phenyl trimethicone (556 Cosmetic Grade Fluid from Dow Corning), or trimethylsiloxyphenyl dimethicone (PDM-20, PDM-200, or PDM-1000 from Wacker-Belsil). Other examples include alkyl dimethicones wherein at least R' is a fatty alkyl (*e.g.,* $C_{12-22}$). A suitable alkyl dimethicone is cetyl dimethicone, wherein R' is a straight C16 chain and R is methyl. Cetyl dimethicone, is available as s 2502 Cosmetic Fluid from Dow Corning or as Abil Wax 9801 or 9814 from Evonik Goldschmidt GmbH.

Cyclic silicones are one type of silicone oil that may be used in the composition. Such silicones have the general formula:

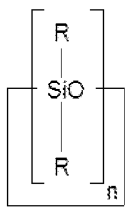

wherein R is independently selected from hydrogen or $C_{1-30}$ straight or branched chain, saturated or unsaturated alkyl, phenyl or aryl, trialkylsiloxy; and where n=3-8 and mixtures thereof. Commonly, a mixture of cyclomethicones is used where n is 4, 5, and/or 6. Commercially available cyclomethicones include Dow Corning UP-1001 Ultra Pure Fluid *(i.e.* n=4), Dow Corning XIAMETER® PMX-0245 (*i.e.* n=5), Dow Corning XIAMETER® PMX-0245 (*i.e.* n=6), Dow Corning 245 fluid (*i.e.* n=4 and 5), and Dow Corning 345 fluid (*i.e.* n=4, 5, and 6).

Suitable hydrocarbon oils include straight, branched, or cyclic alkanes and alkenes. The chain length may be selected based on desired functional characteristics such as volatility. Suitable volatile hydrocarbons may have between 5-20 carbon atoms or, alternately, between 8-16 carbon atoms.

Other suitable oils include esters. The suitable esters typically contained at least 10 carbon atoms. These esters include esters with hydrocarbyl chains derived from fatty acids or alcohols (*e.g.,* mono-esters, polyhydric alcohol esters, and di- and tri-carboxylic acid esters). The hydrocarbyl radicals of the esters hereof may include or have covalently bonded thereto other compatible functionalities, such as amides and alkoxy moieties (*e.g.,* ethoxy or ether linkages, etc.). Exemplary esters include, but are not limited to, isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, C12-15 alkyl benzoate, diisopropyl adipate, dibutyl adipate, and oleyl adipate. Other suitable esters are further described in the Personal Care Product Council's International Cosmetic Ingredient Dictionary and Handbook, Thirteenth Edition, 2010, under the functional category of "Esters." Other esters suitable for use in the sunscreen composition include those known as polyhydric alcohol esters and glycerides.

Other suitable oils include amides. Amides include compounds having an amide functional group while being liquid at 25 °C and insoluble in water. Suitable amides include N-acetyl-N-butylaminopropionate, isopropyl N-lauroylsarcosinate, and N,N,-diethyltoluamide. Other suitable amides are disclosed in U.S. Patent No. 6,872,401.

Other suitable oils include ethers. Suitable ethers include saturated and unsaturated fatty ethers of a polyhydric alcohol, and alkoxylated derivatives thereof. Exemplary ethers include $C_{4-20}$ alkyl ethers of polypropylene glycols, and di-$C_{8-30}$ alkyl ethers. Suitable examples of these materials include PPG-14 butyl ether, PPG-15 stearyl ether, dioctyl ether, dodecyl octyl ether, and mixtures thereof.

The sunscreen composition may comprise an aqueous phase. The sunscreen composition may comprise from about 1% to about 95%, by weight of the composition, aqueous phase. The composition may comprise from about 1%, 3%, 5%, 10%, 15%, 20%, 25%, 30%, 35%, 40%, 45%, 50%, 55%, 60%, 65%, 70%, 75%, 80%, or 90% to about 95%, 90%, 85%, 80%, 75%, 70%, 65%, 60%, 55%, 50%, 45%, 40%, 35%, 30%, 25%, 20%, 15%, 10%, or 5%, by weight of the composition, aqueous phase. The aqueous phase comprise aqueous carrier such as water, water miscible solvents, and other water soluble materials. Suitable water miscible solvents include monohydric alcohols, dihydric alcohols, polyhydric alcohols, glycerol, glycols, polyalkylene glycols such as polyethylene glycol, and mixtures thereof. Particularly suitable solvents, include lower aliphatic alcohols such as ethanol, propanol, butanol, isopropanol; diols such as 1,2-propanediol,1,3-propanediol, butanediol, pentanediol, hexanediol, heptanediol, decanediol; glycerin; water, and mixtures thereof. In certain embodiments, the sunscreen composition comprises water, diols, glycerin, and combinations thereof.

The sunscreen composition may comprise an emulsifier. An emulsifier is particularly suitable when the composition is in the form of an emulsion or if immiscible materials are being combined. The sunscreen composition may comprise from about 0.05%, 0.1%, 0.2%, 0.3%, 0.5%, or 1% to about 20%, 10%, 5%, 3%, 2%, or 1% emulsifier. Emulsifiers may be nonionic, anionic or cationic. Non-limiting examples of emulsifiers are disclosed in U.S. Patent 3,755,560, U.S. Patent 4,421,769, and McCutcheon's, Emulsifiers and Detergents, 2010 Annual Ed., published by M. C. Publishing Co. Other suitable emulsifiers are further described in the Personal Care Product Council's International Cosmetic Ingredient Dictionary and Handbook, Thirteenth Edition, 2006, under the functional category of "Surfactants - Emulsifying Agents."

Suitable emulsifiers include the following classes of ethers and esters: ethers of polyglycols and of fatty alcohols, esters of polyglycols and of fatty acids, ethers of polyglycols and of fatty alcohols which are glycosylated, esters of polyglycols and of fatty acids which are glycosylated, ethers of $C_{12-30}$ alcohols and of glycerol or of polyglycerol, esters of $C_{12-30}$ fatty acids and of glycerol or of polyglycerol, ethers of oxyalkylene-modified $C_{12-30}$ alcohols and of glycerol or polyglycerol, ethers of $C_{12-30}$ fatty alcohols comprising and of sucrose or of glucose, esters of sucrose and of $C_{12-30}$ fatty acids, esters of pentaerythritol and of $C_{12-30}$ fatty acids, esters of sorbitol and/or of sorbitan and of $C_{12-30}$ fatty acids, ethers of sorbitol and/or of sorbitan and of alkoxylated sorbitan, ethers of polyglycols and of cholesterol, esters of $C_{12-30}$ fatty acids and of alkoxylated ethers of sorbitol and/or sorbitan, and combinations thereof.

Linear or branched type silicone emulsifiers may also be used. Particularly useful polyether modified silicones include KF-6011, KF-6012, KF-6013, KF-6015, KF-6015, KF-6017, KF-6043, KF-6028, and KF-6038 from Shin Etsu. Also particularly useful are the polyglycerolated linear or branched siloxane emulsifiers including KF-6100, KF-6104, and KF-6105 from Shin Etsu.

The sunscreen composition may comprise an additional UV active. Additional UV actives include materials that are water soluble such as 2-phenylbenzimidazole-5-sulfonic acid and particulate UV blockers such as inorganic particulates including zinc oxide and titanium dioxide and organic particulates including methylene bis-benzotriazolyl tetramethylbutylphenol (commercially available as Tinosorb® M from BASF). Additional UV actives further include liquid UV actives and solubilized solid UV actives that are not associated with the UV composite (*e.g.,* free liquid UV actives).

The sunscreen composition may comprise a photostabilizer. The composition may comprise from about 0.0001%, 0.001%, 0.01%, 0.05%, 0.1%, 0.5%, or 1% to about 20%, 10%, 7%, or 5%, by weight of the composition, of

one or more suitable photostabilizer.

A suitable photostabilizer is alpha-cyanodiphenylacrylate is as disclosed in U.S. Patent No. 7,713,519. The alpha-cyanodiphenylacrylate may have the general formula:

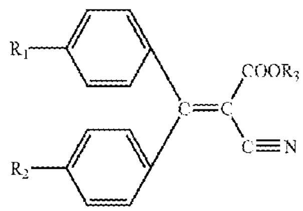

wherein one or both of R1 and R2 is independently a straight or branched chain C1-30 alkoxy radical and any non-alkoxy R1 or R2 radical is hydrogen; and R3 is a straight or branched chain C1-30 alkyl. Alternately, one or both of R1 and R2 is independently a C1-8 alkoxy radical and any non-alkoxy R1 or R2 radical is hydrogen; and R3 is a straight of branched chain C2-20 alkyl. Alternately, one or both of R1 and R2 is independently methoxy, and any non-methoxy R1 or R2 is hydrogen; and R3 is a straight or branched chain C2-20 alkyl.

A suitable alpha-cyanodiphenylacrylate is ethylhexyl methoxycrylene, or 2-ethylhexyl 2-cyano-3-(4-methoxyphenyl)-3-phenylpropenoate, wherein R1 is methoxy, R2 is hydrogen, and R3 is 2-ethylhexyl. This material is available from Hallstar Company under trade name Solastay® S1.

Another suitable photostabilizer includes diesters or polyesters of naphthalene dicarboxylic acid as disclosed in U.S. Patent Nos. 5,993,789, 6,113,931, 6,126,925 and 6,284,916. Suitable diesters or polyesters of naphthalene dicarboxylic acid may have the following formula:

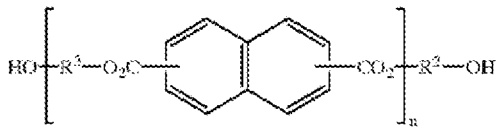

or

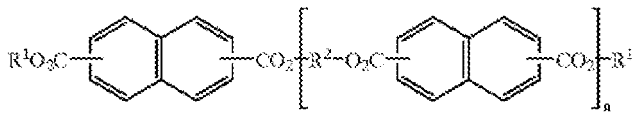

wherein each $R^1$ independently is an alkyl group having 1 to 22 carbon atoms, or a diol having the formula $HO-R^2-OH$, or a polyglycol having the formula $HO-R^3-(-O-R^2-)_m-OH$, and, wherein $R^3$ and $R^3$, same or different, are each an alkylene group, straight chain or branched, having 1 to 6 carbon atoms, wherein m and n are each 1 to about 100, 1 to about 10, or 2 to about 7. A suitable diester of naphthalene dicarboxylic acid is diethylhexyl 2,6-naphthalate available as Corapan® TQ from Symrise.

Another suitable photostabilizer is 4-hydroxybenzylidenemalonate derivatives or 4-hydroxycinnamate derivatives. Suitable materials may have the following formula:

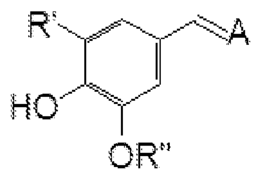

wherein A is a chromophoric group that absorbs UV-radiation, comprises one divalent group or two monovalent groups with at least one group having carbonyl (C=O) functionality; R' is hydrogen, a linear or branched $C_1$-$C_8$ alkyl radical or a linear or branched $C_1$-$C_8$ alkoxy radical; and R" is a linear or branched $C_1$-$C_8$ alkyl radical. Exemplary compounds include ethyl-alpha-cyano-3,5-dimethoxy-4-hydroxy cinnamate, ethyl-alpha-acetyl-3,5-dimethoxy-4-hydroxy cinnamate, iso-propyl-alpha-acetyl-3,5-dimethoxy-4-hydroxy cinnamate, iso-amyl-alpha-acetyl-3,5-dimethoxy-4-hydroxy cinnamate, 2-ethylhexyl-alpha-acetyl-3,5-dimethoxy-4-hydroxy cinnamate, diethyl-3,5-dimethoxy-4-hydroxy benzylidene

malonate, di-(2-ethylhexyl)-3,5-dimethoxy-4-hydroxy benzylidene malonate, diisoamyl-3,5-dimethoxy-4-hydroxy benzylidene malonate, didodecyl-3,5-dimethoxy-4-hydroxy benzylidene malonate, dipalmitoyl-3,5-dimethoxy-4-hydroxy benzylidene malonate, and di-isopropyl-3,5-dimethoxy-4-hydroxy benzylidene malonate. A particularly suitable compound is diethylhexyl syringylidenemalonate (INCI name) available under the tradename Oxynex® ST from EMD Chemicals, Inc. Additional suitable 4-hydroxybenzylidenemalonate derivatives or 4-hydroxycinnamate derivatives are disclosed in U.S. Patent No. 7,357,919 and U.S. Patent Application Publication No. 2003/0108492A1 and US2003/0157035A.

Other suitable photostabilizers include a 2-pyrrolidinone-4-carboxy ester compounds as described in U.S. Patent Application Publication No. 2010/0183529; silicon-containing s-triazines substituted with two aminobenzoate or aminobenzamide groups as described in U.S. Patent Application Publication No. 2008/0145324; fluorene derivatives as described in U.S. Patent Application Publications Nos. 2004/00579912, 2004/00579914, 200/00579916, and 2004/062726; piperidinol salts as described in U.S. Patent Application Publications No. 2005/0220727 including tris(tetramethylhydroxypiperidinol) citrate sold under the tradename Tinogard® Q by Ciba; and arylalkyl amides and esters as described in U.S. Patent Application Publication No. 2008/0019930.

Other suitable photostabilizers are listed in the functional category of "Light Stabilizers" in the Personal Care Product Council’s International Cosmetic Ingredient Dictionary and Handbook, Thirteenth Edition, 2010.

The sunscreen composition may comprise one or more optional components to provide an efficacious and/or consumer desirable product. For example, the composition can include other actives or agents. For instance, suitable optional skin care actives and agents may include an active or agent selected from a group consisting of sugar amines, vitamins, oil control agents, photosterols, hexamidine compounds, tightening agents, anti-wrinkle actives, anti-atrophy actives, retinoids, peptides, particulate materials, anti-cellulite agents, desquamation actives, anti-acne actives, antioxidants, radical scavengers, conditioning agents, anti-inflammatory agents, tanning actives, skin lightening agents, botanical extracts, antimicrobial actives, antifungal actives, antibacterial actives, antiperspirant actives, sensates, preservatives, anti-dandruff actives, detersive surfactants, and combinations thereof. Examples of these materials are provided in U.S. Patent Application Publication No. US2007/0185038A1, US2006/0275237A1, US2004/ 0175347A1, and US2006/0263309A1. The sunscreen composition may comprise from about 0.0001%, 0.001%, 0.01%, 0.05%, 0.1%, 0.5%, 1%, 2%, or 3% to about 30%, 25%, 20%, 15%, 10%, 7%, 5%, 3%, 2%, or 1%, by weight of the composition, of one or more skin care actives.

In certain embodiments, skin care actives may be selected from sugar amines, vitamins, hexamidine compounds, peptides, and combinations thereof.

Examples of sugar amines that are useful herein include glucosamine, N-acetyl glucosamine, mannosamine, N-acetyl mannosamine, galactosamine, N-acetyl galactosamine, their isomers (*e.g.,* stereoisomers), and their salts (*e.g.,* HCl salt). Preferred for use herein are glucosamine, particularly D-glucosamine and N-acetyl glucosamine, particularly N-acetyl-D-glucosamine.

"Vitamins" means vitamins, pro-vitamins, and their salts, isomers and derivatives. Non-limiting examples of suitable vitamins include: vitamin B compounds (including B1 compounds, B2 compounds, B3 compound, B5 compounds, such as panthenol or "pro-B5", pantothenic acid, pantothenyl; B6 compounds, such as pyroxidine, pyridoxal, pyridoxamine; carnitine, thiamine, riboflavin); vitamin A compounds, and all natural and/or synthetic analogs of Vitamin A, including retinoids, retinol, retinyl acetate, retinyl palmitate, retinoic acid, retinaldehyde, retinyl propionate, carotenoids (pro-vitamin A), and other compounds which possess the biological activity of Vitamin A; vitamin D compounds; vitamin K compounds; vitamin E compounds, or tocopherol, including tocopherol sorbate, tocopherol acetate, other esters of tocopherol and tocopheryl compounds; vitamin C compounds, including ascorbate, ascorbyl esters of fatty acids, and ascorbic acid derivatives, for example, ascorbyl phosphates such as magnesium ascorbyl phosphate and sodium ascorbyl phosphate, ascorbyl glucoside, and ascorbyl sorbate; and vitamin F compounds, such as saturated and/or unsaturated fatty acids.

In certain embodiments, the sunscreen compositions comprise a vitamin B3 compound. As used herein, "vitamin B3 compound" means a compound having the formula:

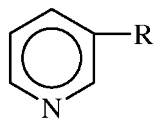

wherein R is - $CONH_2$ (*i.e.,* niacinamide), - COOH (*i.e.,* nicotinic acid) or - CH2OH (*i.e.,* nicotinyl alcohol); derivatives thereof; and salts of any of the foregoing.

The sunscreen compositions may include hexamidine compounds, its salts, and derivatives. As used herein, "hexamidine compound" means a compound having the formula:

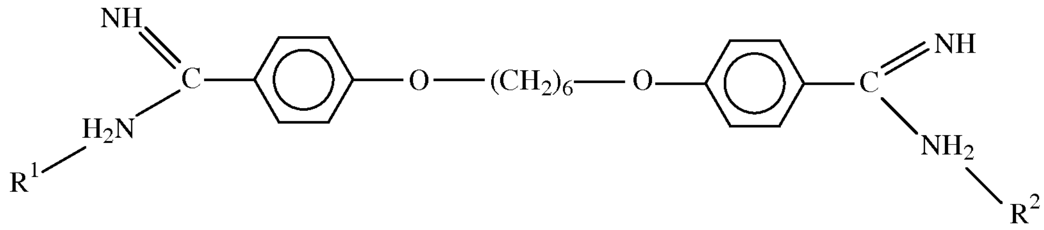

wherein $R^1$ and $R^2$ are optional or are organic acids (*e.g.,* sulfonic acids, etc.) A suitable hexamidine compounds includes hexamidine diisethionate, commercially available as Eleastab® HP100 from Laboratoires Serobiologiques.

As used herein, "peptide" refers to peptides containing ten or fewer amino acids and their derivatives, isomers, and complexes with other species such as metal ions (*e.g.,* copper, zinc, manganese, magnesium, and the like). Peptide refers to both naturally occurring and synthesized peptides. Also useful herein are naturally occurring and commercially available compositions that contain peptides. The peptides may contain at least one basic amino acid (*e.g.,* histidine, lysine, arginine). For example, suitable peptides are the dipeptide carnosine (beta-ala-his), the tripeptide gly-his-lys, the tripeptide his-gly-gly, the tripeptide gly-gly-his, the tripeptide gly-his-gly, the tetrapeptide gly-gln-pro-arg, the pentapeptide lys-thr-thr-lys-ser, lipophilic derivatives of peptides, and metal complexes of the aforementioned (*e.g.,* copper complex of the tripeptide his-gly-gly (also known as Iamin)). Other suitable peptides include Peptide CK (arg-lys-arg); Peptide CK+ (ac-arg-lys-arg-NH2); and Peptide E, arg-ser-arg-lys. A commercially available tripeptide derivative-containing composition is Biopeptide CL® (from Sederma, France), which contains 100 ppm of palmitoyl-gly-his-lys and is commercially available. A commercially available pentapeptide derivative-containing composition is Matrixyl® (from Sederma, France), which contains 100 ppm of palmitoyl-lys-thr-thr-lys-ser. A suitable peptide is a dipeptide based molecule having a C terminal amino acid of threonine, such as plamitoyl-lys-thr, as described in US Patent Application Publication 2007/0020220 A1.

Peptide derivatives useful herein include lipophilic derivatives such as palmitoyl derivatives. In one embodiment, the peptide is selected from palmitoyl-lys-thr-thr-lys-ser, palmitoyl-gly-his-lys, their derivatives, and combinations thereof.

Any other suitable optional component can also be included in the sunscreen composition of the present invention, such as those ingredients that are conventionally used in given product types. The Personal Care Product Council's International Cosmetic Ingredient Dictionary and Handbook, Thirteenth Edition, 2010, describes a wide variety of nonlimiting functional materials that can be added to the composition herein. Examples of these functional classes include, but are not limited to: abrasives, absorbents, fragrances, anti-acne agents, anti-caking agents, antifoaming agents, antimicrobial agents (*e.g.*, iodopropyl butylcarbamate), antifungal agents, antioxidants, binders, buffering agents, bulking agents, chelating agents, colorants, cosmetic astringents, cosmetic biocides, denaturants, drug astringents, external analgesics, film formers, opacifying agents, pH adjusters, plant derivatives, plant extracts, plant tissue extracts, plant seed extracts, plant oils, botanicals, botanical extracts, preservatives, propellants, reducing agents, sebum control agents, sequestrants, skin bleaching agents, skin-conditioning agents (*e.g.* humectants and occlusive agents), and skin protectorants. Other suitable optional personal care ingredients include materials listed in paragraphs 513-839 of U.S Patent Application No. 2010/0112100.

<u>Method of Making the Sunscreen Compositions</u>

As presented above, the sunscreen composition may take a variety of forms. The following methods are exemplary and are not to be read as limiting. The UV composite may be pre-formed by combining the silicone elastomer and the liquid UV active in a suitable vessel. The UV composite may be left for a time period of from several minutes (*e.g.,* 15, 30, or 60 minutes) to several hours (*e.g.,* 12, 24, 36, or 48 hours) to allow the silicone to full adsorption and swelling and during this time mixing may optionally be done by stirring, milling, shaking, or some other mechanical means. The UV composite and the other oil phase materials may be mixed (*e.g.,* magnetic stirrer with spin bar) and optionally heated to 80°C. The oil phase is mixed until homogenous.

When the sunscreen care composition is in the form of an emulsion, the oil phase may be prepared according to the method above. In a separate vessel, the aqueous phase may be prepared by combining the aqueous carrier such as water and/or a water miscible solvent with any water soluble materials, if present. The combination may be mixed (*e.g.,* magnetic stirrer with spin bar) and optionally heated to 80°C. Depending upon the particular emulsion form (O/W or W/O) an emulsifier may be added to the suitable phase. Typically, the emulsifier may be added to the continuous phase. Again, depending upon the desired emulsion form, the oil phase may be added to the aqueous phase or vice versa. The emulsion may be mixed (*e.g.,* magnetic stirrer with spin bar, rotor-stator mill, propeller type mixer, etc.). The composition is mixed until homogenous. The emulsion may be transferred to an acceptable container. The emulsion may be cooled.

Examples of UV Composites

The following are exemplary silicone elastomers and liquid UV active combinations. In several of the examples, a UV composite forms. The following silicone elastomers were used in the examples:

1. Dow Corning® 9045 - dimethicone crosspolymer supplied in cyclomethicone (D5).
2. Velvesil 125 - C30-45 alkyl cetearyl dimethicone crosspolymer supplied in cyclomethicone (D5).
3. KSG-42 - vinyl dimethicone / lauryl dimethicone crosspolymer supplied in isododecane.
4. Dow Corning® EL-8050 ID SOEB - dimethicone / bis-isobutyl PPG 20 crosspolymer supplied in isododecane.
5. KSG-240 - dimethicone / PEG-10 crosspolymer supplier in cyclomethicone (D5).
6. KSG-320 - PEG-15 / lauryl dimethicone crosspolymer supplied in isododecane.
7. KSG-820 - lauryl dimethicone / poly-glycerin-3 crosspolymer supplied in isododecane.

The silicone elastomers are supplied in a non-UV solvent. The solvent was extracted and the silicone elastomer deswollen by solvent extraction techniques described above. Observations of the deswollen elastomer were taken prior to re-swelling with the liquid UV active. Various ratios of liquid UV active to silicone elastomer were prepared. The samples were allowed to swell for approximately 2-3 hours at ambient conditions with mechanical agitation (approximately 1100 vibrations per minute) after which a visual inspection was performed.

Table 1 demonstrates the loading capacity of octisalate (2-ethylhexyl-salicylate) in various silicone elastomers (example numbers correspond to the elastomers enumerated above). In several examples, UV composites were formed with no decantable liquid UV active at ratios of 1:1 and higher. For instance, the dimethicone / PEG-10 crosspolymer (KSG-240) of Example A5 was able to form a UV composite at ratios from 1:1 to 4:1. The silicone elastomer exceeded capacity at the ratio of 5:1. Therefore, the maximum liquid UV load (and more specifically octisalate load) is between 4:1 and 5:1. Examples with "*" indicate that free liquid was observed at the specified ratio.

| | **Observations / Ratio of Octisalate:Silicone Elastomer** | | | | | | | | |
|---|---|---|---|---|---|---|---|---|---|
| **Ex.** | **0:1 (no octisalate)** | **1:1** | **2:1** | **3:1** | **4:1** | **5:1** | **6:1** | **8:1** | **10:1** |
| A1 | white, rubbery material | ** Slurry of gel-like particles in liquid* | ** Thin slurry of gel-like particles, lots of liquid* | ---- | ---- | ---- | ---- | ---- | ---- |
| A2 | rubbery non-cohesive particles, lot of static | gel-like particles | ** Gel-like particles / mass, slight free liquid* | ---- | ---- | ---- | ---- | ---- | ---- |
| A3 | white, firm, rubbery material | gel-like particles | gelled mass | ** Slurry of gel-like particles* | ---- | ---- | ---- | ---- | ---- |
| A4 | yellow, slight sticky, rubbery material | yellowish, gel-like particles | yellowish gel-like particles | ** Gel-like particles, slight liquid* | ---- | ---- | ---- | ---- | ---- |
| A5 | very sticky, rubbery material | gel-like particles | gel-like particles | gel-like particles | gel-like mass | ** Slurry of gel-like particles* | ---- | ---- | ---- |
| A6 | white, sticky, rubbery material | gel-like particles | gel-like particles | gel-like particles | gel-like particles | gel-like particles | gel-like particles | loose gel-like mass | ** Slurry of gel-like particles* |
| A7 | white, firm, rubbery material | gel-like particles | gel-like particles | gel-like particles | ** Slurry of gel-like particles* | ---- | ---- | ---- | ---- |

**Table 1**

Table 2 demonstrates the loading capacity of octinoxate (octyl methoxycinnamate) in various silicone elastomers (example numbers correspond to the elastomers enumerated above). In several examples, UV composites were formed with no decantable liquid UV active at ratios of 1:1 and higher. For instance, the dimethicone / PEG-10 crosspolymer (KSG-240) of Example B5 was able to form a UV composite at ratios from 1:1 to 2:1. The silicone elastomer exceeded capacity at the ratio of 3:1. Therefore, the maximum liquid UV load (and more specifically octinoxate load) is between 2:1 and 3:1. Examples with "*" indicate that free liquid was observed at the specified ratio.

| | **Observations / Ratio of Octinoxate:Silicone Elastomer** | | | | |
|---|---|---|---|---|---|
| **Ex.** | **0:1 (no octinoxate)** | **1:1** | **2:1** | **3:1** | **4:1** |
| B1 | white, rubbery material | - * *Slurry of gel-like particles in liquid* | * *Thin slurry of gel-like particles, lots of liquid* | ---- | ---- |
| B2 | rubbery non-cohesive particles, lot of static | gel-like particles | * *Slurry of gel-like particles (slight liquid)* | ---- | ---- |
| B3 | white, firm, rubbery material | gel-like particles | Gelled mass | - * *Slurry of gel-like particles ----* | ---- |
| B4 | yellow, slight sticky, rubbery material | yellowish, cream-like mass | - * *Yellow slurry of gel-like particles* | ---- | ---- |
| B5 | very sticky, rubbery material | gel like particles | gel-like particles | - * *Slurry of gel-like particles* | ---- |
| B6 | white, sticky, rubbery material | gel like particles | gel-like particles | gel-like mass / particles | - * *Gel-like particles / mass, slight liquid* |
| B7 | white, firm, rubbery material | translucent, gel-like mass (a few particles) | - * *Slurry of gel-like particles (slight liquid)* | ---- | ---- |

**Table 2**

Table 3 demonstrates the loading capacity of a mixed liquid UV system in various silicone elastomers (example numbers correspond to the elastomers enumerated above). The mixed liquid UV system includes both liquid and solubilized solid UV active. The mixed liquid UV system includes 3 parts avobenzone, 6 parts oxybenzone, 15 parts homosalate, 2.6 parts octocrylane, and 5 parts octisalate; all by weight. In several examples, UV composites were formed with no decantable liquid UV active at ratios of 1:1 and higher. For instance, the PEG-15 / lauryl dimethicone crosspolymer (KSG-320) of Example C6 was able to form a UV composite at ratios from 1:1 to 5:1. The silicone elastomer exceeded capacity at the ratio of 6:1. Therefore, the maximum mixed liquid UV system load is between 5:1 and 6:1. Examples with "*" indicate that free liquid was observed at the specified ratio.

| | **Observations / Ratio of Mixed Liquid UV System:Silicone Elastomer** | | | | | | |
|---|---|---|---|---|---|---|---|
| **Ex.** | **0:1 (no mixed UV)** | **1:1** | **2:1** | **3:1** | **4:1** | **5:1** | **6:1** |
| C1 | white, rubbery material | - * *Slurry of gel-like particles in liquid* | * *Thin slurry of gel-like particles, lots of liquid* | ---- | ---- | ---- | ---- |

(continued)

| | Observations / Ratio of Mixed Liquid UV System:Silicone Elastomer | | | | | | |
|---|---|---|---|---|---|---|---|
| **Ex.** | **0:1 (no mixed UV)** | **1:1** | **2:1** | **3:1** | **4:1** | **5:1** | **6:1** |
| C3 | white, firm, rubbery material | gel-like particles | - * *Slurry of gel-like particles in* | * *Thin slurry of gel-like particles, lots* | ---- | ---- | ---- |
| | | | *liquid* | *of liquid* | | | |
| C4 | yellow, slight sticky, rubbery material | gel-like particles | * *Gel-like particles / mass, slight liquid* | - * *Slurry of gel-like particles* | ---- | ---- | ---- |
| C6 | yellow, sticky, rubbery material | gel-like particles | gel-like particles | gel-like particles/mass | gel-like mass | gel-like mass | * *Thick slurry of gel-like particles* |

**Table 3**

SPF Efficacy

In vitro UV efficacy data was collected on select UV composites as described in Table 2 and a control leg with liquid UV active, octinoxate, alone. The in vivo SPF of each example was measured by applying each composition to roughened PMMA plates (HD6 plates from Helioscience, Marseille, France), allowing the applied product to set for 15 minutes, and then measuring the in vitro SPF of the resulting product film using a Labsphere UV2000 (Labsphere, Inc., North Sutton, NH). The examples comprise the UV composite comprising the silicone elastomer and octinoxate which are applied to ensure a consistent amount (0.0100g) of octinoxate is present in each example as to the control. The results, as provided in Table 4, demonstrate that the UV composites yield higher in vitro SPF values than the neat octinoxate of the control. The UV composites are providing enhanced UV absorption efficacy (via a boost in SPF) as compared to the UV liquid alone. The examples comprise a slight amount of excess liquid UV active, but the SPF enhancement beyond the control is clearly a result of the UV composite. It is noted that one skilled in the art would recognized that the silicone elastomers used in the present UV composites have no significant UV efficacy alone.

| Ex. | Octinoxate:Elastomer Ratio | Amount Applied (g) | Octinoxate Amount Applied (g) | In Vitro SPF |
|---|---|---|---|---|
| Control | 1:0 | 0.0100 | 0.0100 | 9.1 |
| B3 | 3:1 | 0.0133 | 0.0100 | 13.6 |
| B4 | 2:1 | 0.0150 | 0.0100 | 12.7 |
| B7 | 2:1 | 0.0150 | 0.0100 | 16.8 |

**Table 4**

Sunscreen Composition Examples

The following are examples of sunscreen compositions comprising exemplary UV composites as described in Tables 1, 2, and 3.

| | Comp. 1 | Comp. 2 | Comp. 3 | Comp. 4 | Comp. 5 | Comp. 6 |
|---|---|---|---|---|---|---|
| Water Phase: | | | | | | |
| Water | qs | qs | qs | qs | qs | qs |
| Glycerin | 5.0 | 10.0 | 1.0 | 5.0 | 5.0 | 5.0 |
| Disodium EDTA | 0.1 | 0.1 | 0.1 | 0.1 | 0.1 | 0.1 |

(continued)

| | Comp. 1 | Comp. 2 | Comp. 3 | Comp. 4 | Comp. 5 | Comp. 6 |
|---|---|---|---|---|---|---|
| Water Phase: | | | | | | |
| Methylparaben | 0.2 | 0.2 | 0.2 | 0.2 | 0.2 | 0.2 |
| Niacinamide | 4.0 | 2.0 | 1.0 | 1.0 | 4.0 | 4.0 |
| D-panthenol | 0.5 | 0.25 | 0.5 | 0.5 | 2.0 | 0.5 |
| Phenylbenzimidazole Sulfonic Acid | 1.0 | -- | 1.0 | -- | -- | -- |
| Pentylene Glycol | 1.0 | 1.0 | 1.0 | 1.0 | 1.0 | 1.0 |
| Benzyl alcohol | 0.25 | 0.25 | 0.25 | 0.25 | 0.25 | 0.25 |
| Triethanolamine | 0.64 | -- | 0.64 | -- | -- | -- |
| Oil Phase: | | | | | | |
| Example A3 (2:1)[1] | 7.5 | -- | -- | -- | -- | -- |
| Example A8 (3:1) | -- | 6.5 | -- | -- | -- | -- |
| Example B4 (1:1) | -- | -- | 15.0 | -- | -- | -- |
| Example B8 (1:1) | -- | -- | -- | 8.0 | -- | -- |
| Example C3 (1:1) | -- | -- | -- | -- | 10.0 | -- |
| Example C4 (1:1) | -- | -- | -- | -- | -- | 15.0 |
| Isopropyl Isostearate | -- | 1.0 | -- | 3.0 | -- | 2.0 |
| Octocrylene | -- | 3.0 | -- | 5.0 | -- | -- |
| Vitamin E Acetate | 0.1 | 0.5 | 0.5 | 1.0 | 0.1 | 0.1 |
| Ethylparaben | 0.2 | 0.2 | 0.2 | 0.2 | 0.2 | 0.2 |
| Propylparaben | 0.2 | 0.2 | 0.2 | 0.2 | 0.2 | 0.2 |
| Cetyl alcohol | 1.0 | 0.3 | 1.0 | 0.3 | 0.3 | 1.0 |
| Stearyl alcohol | 1.0 | 0.4 | 1.0 | 0.4 | 0.4 | 1.0 |
| Behenyl alcohol | 1.0 | 0.4 | 1.0 | 0.4 | 0.4 | 1.0 |
| Cetearyl Glucoside/ Cetearyl Alcohol[2] | 0.3 | 0.3 | 0.3 | 0.3 | 0.3 | 0.3 |
| PEG-100 stearate | 0.3 | 0.3 | 0.3 | 0.3 | 0.3 | 0.3 |
| Tinosorb S[5] | -- | 1.0 | -- | 1.5 | -- | -- |
| Thickener: | | | | | | |
| Sepigel™ 305[3] | 3.5 | 2.5 | 3.5 | 3.0 | 2.5 | 2.5 |
| Additional Ineredients: | | | | | | |
| Microthene FN510[4] | 2. | 1.0 | 1.0 | -- | -- | 1.0 |
| Dow Corning™ 1503[5] | 2.0 | -- | 0.5 | 2.0 | 2.0 | 0.5 |
| Total: | 100% | 100% | 100% | 100% | 100% | 100% |

[1] Weight percent of UV composite at the listed ratio of liquid UV active to silicone elastomer.
[2] Emulgade™ PL68/50 from Cognis
[3] Bis-Ethylhexyloxyphenol Methoxyphenyl Triazine from BASF
[4] Polyacrylamide, C13-14 isoparaffin, and laureth-7 from Seppic
[5] Polyethylene homopolymer spheres from Equistar
[6] Dimethicone and dimethiconol from Dow Corning

In a suitable vessel, the water phase ingredients are combined and heated to 75°C. In a separate suitable

vessel, the oil phase ingredients are combined and heated to 75°C. Next the oil phase is added to the water phase and the resulting emulsion is milled (*e.g.,* with a rotor-stator mill). The thickener is then added to the emulsion and the emulsion is cooled to 45°C while stirring. At 45°C, the remaining additional ingredients are added. The product is then cooled with stirring to 30°C, milled again, and then poured into suitable containers.

Sunscreen Compositions Demonstrating Feel Benefit of UV Composite

In order to demonstrate the superior formulation results achieved by use of the UV composite, two sunscreen compositions were prepared, one using the UV composite and one using the same level of UV active and silicone elastomer but prepared using commercially available silicone elastomer with the accompanying non-UV solvent.

| | Comp. Alpha | Comp. Beta |
|---|---|---|
| Water Phase: | | |
| Water | qs | qs |
| Glycerin | 5.0 | 5.0 |
| Disodium EDTA | 0.1 | 0.1 |
| Glydant Plus Liquid[1] | 0.3 | 0.3 |
| Polysorbate 20 | 0.2 | 0.2 |
| Oil Phase: | | |
| Example A4 [2] | 6.66 | -- |
| EL-8051 IN [3] | -- | 13.84 |
| Octisalate | -- | 5.0 |
| Thickener: | | |
| Sepigel™ 305 [4] | 2.5 | 2.5 |
| Total: | 100% | 100% |

[1] DMDM Hydantoin, Iodopropynyl butylcarbamate from Lonza
[2] A 3:1 blend of octisalate to dimethicone / bis-isobutyl PPG 20 crosspolymer, which consists of UV composite of octisalate and the silicone elastomer, with a slight excess of octisalate. Therefore, composition alpha comprises about 5% octisalate and about 1,66% dimethicone / bis-isobutyl PPG 20 crosspolymer,
[3] EL-8051 IN (Dow Corning, Midland, MI) is a commercially available dimethicone / bis-isobutyl PPG 20 crosspolymer supplied as 12% elastomer swollen in isodecyl neopentanoate solvent. In order for composition beta to include the same amount of both octisalate (5%) and dimethicone / bis-isobutyl PPG 20 crosspolymer (1.66%) as composition alpha, 5% octisalate must be added and the EL-8051 IN must be added at a level of 13.84%. As a result, composition beta also contains an additional 12.18% of isodecyl neopentanoate solvent,
[4] Polyacrylamide, C13-14 isoparaffin, and laureth-7 from Seppic™

To provide an equivalent composition (*i.e.,* same weight percentage of octisalate and silicone elastomer) to composition alpha with the UV composite, composition beta with conventional materials would necessarily require the inclusion of about 12.18% of isodecyl neopentanoate. The inclusion of 12.18% isodecyl neopentanoate reduces the available space in the composition for other more efficacious materials such as skin care actives, emollients, or more UV actives. Additionally, isodecyl neopentanoate is an oily material, so it has a negative impact on the skin feel of the formulation. This was demonstrated by conducting a study in which we asked 5 people to feel both products and select which product they thought was less greasy and oily on their skin. Panelists applied a controlled dose (0.1 g) of each product on their volar forearms, rubbed the product into their skin, and were asked to identify which composition was less greasy and oily. All panelists (five out of five) selected composition alpha as being less greasy and oily on their skin.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A sunscreen composition in the form of an emulsion comprising:

   a. from 2% to 40% of a UV composite comprising from 7% to 50%, by weight of the UV composites, of a silicone elastomer swollen in a liquid UV active such that the ratio of liquid UV active to silicone elastomer is from 1:1 to 8:1;
   b. from 3% to 50% of an oil phase, wherein the liquid UV active comprises at least 60% by weight of the oil phase; and
   c. from 40 to 90% of an aqueous phase.

2. The sunscreen composition of Claim 1 wherein the silicone elastomer is an organically modified silicone elastomer.

3. The sunscreen composition of Claim 1 wherein the silicone elastomer is selected from a group consisting of polyorganosiloxane crosspolymer, polyoxyalkylene modified polyorganosiloxane crosspolymer, and polyglycerin modified polyorganosiloxane crosspolymer, preferably the silicone elastomer is selected from a group consisting of alkyl dimethicone/polyglycerin crosspolymer, dimethicone/polyglycerin crosspolymer, dimethicone/poly(propylene glycol) crosspolymer, dimethicone/poly(ethylene glycol) crosspolymer, alkyl dimethicone/poly(propylene glycol) crosspolymer, alkyl dimethicone/poly(ethylene glycol) crosspolymer, and alkyl dimethicone crosspolymer.

4. The sunscreen composition of Claim 1 wherein the silicone elastomer is non-spherical.

5. The sunscreen composition of Claim 1, wherein the sunscreen composition further comprises from 0.01 to 10% of an emulsifier, preferably the emulsifier is selected from a group consisting of ethers of glycerol, polyglycerol, sucrose, glucose, or sorbitol; esters of glycerol, polyglycerol, sucrose, glucose, or sorbitol; and mixtures thereof.

6. The sunscreen composition of Claim 1 wherein the liquid UV active is selected from a group consisting of 2-ethylhexyl-p-methoxycinnamate, homomethyl salicylate, octyldimethyl-p-aminobenzoic acid, 2-ethylhexyl-2-cyano-3,3-diphenylacrylate, 2-ethylhexyl-salicylate, 3,3,5-tri-methylcyclohexylsalicylate, methyl anthranilate, and combinations thereof.

7. The sunscreen composition of Claim 1 wherein the liquid UV active is selected from a group consisting of 2-ethylhexyl salicylate, 2-ethylhexyl-p-methoxycinnamate, and combinations thereof.

8. The sunscreen composition of Claim 1 which comprises a solid UV active selected from a group consisting of dibenzoylmethane compounds, bis-ethylhexyloxyphenol methoxyphenyl triazine, 2,4,6-trianilino-(p-carbo-2'-ethylhexyl-1'-oxy)-1,3,5-triazine, diethylhexyl butamido triazone, diethylamino hydroxybenzoyl hexyl benzoate, benzophenone-3, 4-methylbenzylidene camphor, ethylhexyl bis-isopentylbenzoxazolylphenyl melamine, and combinations thereof, wherein the dibenzoylmethane compound is preferably 4-tert-butyl-4'-methoxy dibenzoylmethane.

9. The sunscreen composition of Claim 1 further comprising a photo stabilizer, which is preferably selected from the group consisting of methoxycrylene, diethylhexyl 2,6-naphthalate, diethylhexyl syringylidenemalonate, and combinations thereof.

10. The sunscreen composition of Claim 1 further comprising an active or agent selected from a group consisting of sugar amines, vitamins, oil control agents, photosterols, hexamidine compounds, tightening agents, anti-wrinkle actives, anti-atrophy actives, flavonoids, N-acyl amino acid compounds, retinoids, peptides, particulate materials, anti-cellulite agents, desquamation actives, anti-acne actives, anti-oxidants, radical scavengers, conditioning agents, anti-inflammatory agents, tanning actives, skin lightening agents, botanical extracts, antimicrobial actives, antifungal actives, antibacterial actives, antiperspirant actives, sensates, preservatives, anti-dandruff actives, substantivity polymers, detersive surfactants, and combinations thereof.

11. A SPF enhancing UV composite for addition to a sunscreen composition, said UV composite comprising a silicone elastomer swollen in a liquid UV active such that the ratio of liquid UV active to silicone elastomer is from 1:1 to 8:1, preferably the UV composite consists essentially of the silicone elastomer swollen in the liquid UV active, and more preferably the silicone elastomer is an organically modified silicone elastomer.

12. The UV composite of Claim 11 wherein the silicone elastomer is selected from a group consisting of polyorganosiloxane crosspolymer, polyoxyalkylene modified polyorganosiloxane crosspolymer, and polyglycerin modified polyorganosiloxane crosspolymer.

**13.** The UV composite of Claim 11 wherein the silicone elastomer is selected from a group consisting of alkyl dimethicone/polyglycerin crosspolymer, dimethicone/polyglycerin crosspolymer, dimethicone/poly(propylene glycol) crosspolymer, dimethicone/poly (ethylene glycol) crosspolymer, alkyl dimethicone/poly(propylene glycol) crosspolymer, alkyl dimethicone/poly(ethylene glycol) crosspolymer, and alkyl dimethicone crosspolymer.

**14.** The UV composite of Claim 11 wherein the liquid UV active is selected from a group consisting of 2-ethylhexyl-p-methoxycinnamate, homomethyl salicylate, octyldimethyl-p-aminobenzoic acid, 2-ethylhexyl-2-cyano-3,3-diphenylacrylate, 2-ethylhexyl-salicylate, 3,3,5-tri-methylcyclohexylsalicylate, methyl anthranilate, and combinations thereof, preferably the liquid UV active is selected from a group consisting of 2-ethylhexyl salicylate, 2-ethylhexyl-p-methoxycinnamate, and combinations thereof, and more preferably, the liquid UV active comprises a solubilized solid UV active.

**15.** The UV composite of Claim 14 wherein the solid UV active is selected from a group consisting of dibenzoylmethane compounds, bis-ethylhexyloxyphenol methoxyphenyl triazine, 2,4,6-trianilino-(p-carbo-2’-ethylhexyl-1’-oxy)-1,3,5-triazine, diethylhexyl butamido triazone, diethylamino hydroxybenzoyl hexyl benzoate, benzophenone-3, 4-methylbenzylidene camphor, ethylhexyl bis-isopentylbenzoxazolylphenyl melamine, and combinations thereof, preferably the dibenzoylmethane compound is 4-tert-butyl-4’-methoxy dibenzoylmethane.

## Patentansprüche

**1.** Sonnenschutzzusammensetzung in der Form einer Emulsion, umfassend:

a. von bis 4 ein UV-Gemisch, das, bezogen auf das Gewicht der UV-Gemische, von bis 5 ein Silikonelastomer umfasst, das in einem flüssigen UV-Wirkstoff gequollen ist, dass das Verhältnis von flüssigem UV-Wirkstoff zu Silikonelastomer von 1 1 bis 8 1 beträgt;
b. von bis 5 eine Ölphase, wobei der flüssige UV-Wirkstoff mindestens 60 Gew.-% der Ölphase umfasst; und
c. von 40 bis 9 eine wässrige Phase.

**2.** Sonnenschutzzusammensetzung nach Anspruch 1, wobei das Silikonelastomer ein organisch modifiziertes Silikonelastomer ist.

**3.** Sonnenschutzzusammensetzung nach Anspruch 1, wobei das Silikonelastomer ausgewählt ist aus einer Gruppe bestehend aus Polyorganosiloxan-Crosspolymer, polyoxyalkylenmodifiziertem Polyorganosiloxan-Crosspolymer und polyglycerinmodifiziertem Polyorganosiloxan-Crosspolymer, wobei das Silikonelastomer vorzugsweise ausgewählt ist aus einer Gruppe bestehend aus Alkyldimethicon/Polyglycerin-Crosspolymer, Dimethicon/Polyglycerin-Crosspolymer, Dimethicon/Poly(propylenglycol)-Crosspolymer, Dimethicon/Poly(ethylenglycol)-Crosspolymer, Alkyldimethicon/Poly(propylenglycol)-Crosspolymer, Alkyldimethicon/Poly(ethylenglycol)-Crosspolymer und Alkyldimethicon-Crosspolymer.

**4.** Sonnenschutzzusammensetzung gemäß Anspruch 1, wobei das Silikonelastomer nicht sphärisch ist.

**5.** Sonnenschutzzusammensetzung nach Anspruch 1, wobei die Sonnenschutzzusammensetzung ferner von bis 1 einen Emulgator umfasst, wobei der Emulgator vorzugsweise ausgewählt ist aus einer Gruppe bestehend aus Ethern von Glycerin, Polyglycerin, Saccharose, Glucose oder Sorbit; Estern von Glycerin, Polyglycerin, Saccharose, Glucose oder Sorbit; und Mischungen davon.

**6.** Sonnenschutzzusammensetzung nach Anspruch 1, wobei der flüssige UV-Wirkstoff ausgewählt ist aus einer Gruppe bestehend aus 2-Ethylhexyl-p-methoxycinnamat, Homomethylsalicylat, Octyldimethyl-p-aminobenzoesäure, 2-Ethylhexyl-2-cyano- -diphenylacrylat, 2-Ethylhexylsalicylat, ,5-Trimethylcyclohexylsalicylat, Methylanthranilat und Kombinationen davon.

**7.** Sonnenschutzzusammensetzung nach Anspruch 1, wobei der flüssige UV-Wirkstoff ausgewählt ist aus einer Gruppe bestehend aus 2-Ethylhexylsalicylat, 2-Ethylhexyl-p-methoxycinnamat und Kombinationen davon.

**8.** Sonnenschutzzusammensetzung nach Anspruch 1, die einen festen UV-Wirkstoff umfasst, der ausgewählt ist aus

einer Gruppe bestehend aus Dibenzoylmethanverbindungen, Bisethylhexyloxyphenolmethoxyphenyltriazin, ,6-Trianilino-(p-carbo-2'-ethylhexyl-1'-oxy)- ,5-thiazin, Diethylhexylbutamidotriazon, Diethylaminohydroxybenzoylhexylbenzoat, Benzophenon-3, 4-Methylbenzylidencampher, Ethylhexylbisisopentylbenzoxazolylphenylmelamin und Kombinationen davon, wobei die Dibenzoylmethanverbindung 4-tert-Butyl-4'-methoxydibenzoylmethan ist.

**9.** Sonnenschutzzusammensetzung nach Anspruch 1, ferner einen Photostabilisator umfassend, der vorzugsweise ausgewählt ist aus einer Gruppe bestehend aus Methoxycrylen, Diethylhexyl- -naphthalat, Diethylhexylsyringylidenmalonat und Kombinationen davon.

**10.** Sonnenschutzzusammensetzung nach Anspruch 1, ferner einen Wirkstoff oder ein Mittel umfassend, das ausgewählt ist aus einer Gruppe bestehend aus Zuckeraminen, Vitaminen, Fettregulierungsmittel, Photosterolen, Hexamidinverbindungen, Straffungsmitteln, faltenbekämpfenden Wirkstoffen, atrophiebekämpfenden Wirkstoffen, Flavonoiden, N-Acylaminosäureverbindungen, Retinoiden, Peptiden, teilchenförmigen Materialien, Anticellulitemitteln, Desquamationswirkstoffen, aknebekämpfenden Wirkstoffen, Antioxidantien, Radikalfängern, Konditionierungsmitteln, Entzündungshemmern, bräunenden Wirkstoffen, Hautaufhellern, botanischen Extrakten, antimikrobiellen Wirkstoffen, pilzbekämpfenden Wirkstoffen, antibakteriellen Wirkstoffen, schweißhemmenden Wirkstoffen, die Sinne ansprechenden Substanzen, Konservierungsmitteln, schuppenbekämpfenden Wirkstoffen, Substantivitätspolymeren, reinigungsaktiven Tensiden und Kombinationen davon.

**11.** LSF-verstärkendes UV-Gemisch zur Zugabe zu einer Sonnenschutzzusammensetzung, wobei das UV-Gemisch ein Silikonelastomer umfasst, das in einem flüssigen UV-Wirkstoff gequollen ist, dass das Verhältnis von flüssigem UV-Wirkstoff zu Silikonelastomer von 1 1 bis 8 1 beträgt, wobei das UV-Gemisch vorzugsweise im Wesentlichen aus dem Silikonelastomer besteht, das in dem flüssigen UV-Wirkstoff gequollen ist, und wobei das Silikonelastomer mehr bevorzugt ein organisch modifiziertes Silikonelastomer ist.

**12.** UV-Gemisch nach Anspruch 11, wobei das Silikonelastomer ausgewählt ist aus einer Gruppe bestehend aus Polyorganosiloxan-Crosspolymer, polyoxyalkylenmodifiziertem Polyorganosiloxan-Crosspolymer und polyglycerinmodifiziertem Polyorganosiloxan-Crosspolymer.

**13.** UV-Gemisch nach Anspruch 11, wobei das Silikonelastomer ausgewählt ist aus einer Gruppe bestehend aus Alkyldimethicon/Polyglycerin-Crosspolymer, Dimethicon/Polyglycerin-Crosspolymer, Dimethicon/Poly(propylenglycol)-Crosspolymer, Dimethicon/Poly(ethylenglycol)-Crosspolymer, Alkyldimethicon/Poly(propylenglycol)-Crosspolymer, Alkyldimethicon/Poly(ethylenglycol)-Crosspolymer und Alkyldimethicon-Crosspolymer.

**14.** UV-Gemisch nach Anspruch 11, wobei der flüssige UV-Wirkstoff ausgewählt ist aus einer Gruppe bestehend aus 2-Ethylhexyl-p-methoxycinnamat, Homomethylsalicylat, Octyldimethyl-p-aminobenzoesäure, 2-Ethylhexyl-2-cyano- - diphenylacrylat, 2-Ethylhexylsalicylat, ,5-Trimethylcyclohexylsalicylat, Methylanthranilat und Kombinationen davon, wobei der flüssige UV-Wirkstoff vorzugsweise ausgewählt ist aus einer Gruppe bestehend aus 2-Ethylhexylsalicylat, 2-Ethylhexyl-p-methoxycinnamat und Kombinationen davon, und wobei der flüssige UV-Wirkstoff mehr bevorzugt einen gelösten festen UV-Wirkstoff umfasst.

**15.** UV-Gemisch nach Anspruch 14, wobei der feste UV-Wirkstoff ausgewählt ist aus einer Gruppe bestehend aus Dibenzoylmethanverbindungen, Bisethylhexyloxyphenolmethoxyphenyltriazin, ,6-Trianilino-(p-carbo-2'-ethylhexyl-1'-oxy)- ,5-triazin, Diethylhexylbutamidotriazon, Diethylaminohydroxybenzoylhexylbenzoat, Benzophenon-3, 4-Methylbenzylidencampher, Ethylhexylbisisopentylbenzoxazolylphenylmelamin und Kombinationen davon, wobei die Dibenzoylmethanverbindung vorzugsweise 4-tert-Butyl-4'-methoxydibenzoylmethan ist.

## Revendications

**1.** Composition d'écran solaire sous la forme d'une émulsion comprenant :

a. de 2 % à 40 % d'un composite UV comprenant de 7 % à 50 % en poids des composites UV, d'un élastomère de silicone gonflé dans un agent actif aux UV liquide de telle sorte que le rapport de l'agent actif aux UV liquide à l'élastomère de silicone va de 1:1 à 8:1 ;
b. de 3 % à 50 % d'une phase huileuse, dans laquelle l'agent actif aux UV liquide constitue au moins 60 % en poids de la phase huileuse ; et
c. de 40 à 90 % d'une phase aqueuse.

2. Composition d'écran solaire selon la revendication 1, dans laquelle l'élastomère de silicone est un élastomère de silicone organiquement modifié.

3. Composition d'écran solaire selon la revendication 1, dans laquelle l'élastomère de silicone est choisi parmi un groupe constitué de polymère réticulé de polyorganosiloxane, polymère réticulé de polyorganosiloxane à modification polyoxyalkylène et polymère réticulé de polyorganosiloxane à modification polyglycérine, de préférence l'élastomère de silicone est choisi parmi un groupe constitué de polymère réticulé alkyldiméthicone/polyglycérine, polymère réticulé diméthicone/polyglycérine, polymère réticulé diméthicone/poly(propylène glycol), polymère réticulé diméthicone/poly(éthylène glycol), polymère réticulé alkyldiméthicone/poly(propylène glycol), polymère réticulé alkyldiméthicone/poly(éthylène glycol) et polymère réticulé alkyldiméthicone.

4. Composition d'écran solaire selon la revendication 1, dans laquelle l'élastomère de silicone est non sphérique.

5. Composition d'écran solaire selon la revendication 1, où la composition d'écran solaire comprend en outre de 0,01 à 10 % d'un émulsifiant, de préférence l'émulsifiant est choisi parmi un groupe constitué d'éthers de glycérol, polyglycérol, saccharose, glucose ou sorbitol ; esters de glycérol, polyglycérol, saccharose, glucose ou sorbitol ; et des mélanges de ceux-ci.

6. Composition d'écran solaire selon la revendication 1, dans laquelle l'agent actif aux UV liquide est choisi parmi un groupe constitué de 2-éthylhexyl-p-méthoxycinnamate, salicylate d'homométhyle, acide octyldiméthyl-p-aminobenzoïque, 2-éthylhexyl-2-cyano-3,3-diphénylacrylate, 2-éthylhexyl-salicylate, 3,3,5-tri-méthylcclohexylsalicylate, anthranilate de méthyle, et leurs combinaisons.

7. Composition d'écran solaire selon la revendication 1, dans laquelle l'agent actif aux UV liquide est choisi parmi un groupe constitué de salicylate de 2-éthylhexyle, 2-éthylhexyl-p-méthoxycinnamate, et leurs combinaisons.

8. Composition d'écran solaire selon la revendication 1, qui comprend un agent actif aux UV solide choisi dans un groupe constitué de composés de dibenzoylméthane, bis-éthylhexyloxyphénol méthoxyphényl triazine, 2,4,6-trianilino-(p-carbo-2'-éthylhexyl-1'-oxy)-1,3,5-triazine, diéthylhexyl butamido triazone, benzoate de diéthylamino hydroxybenzoyl hexyle, benzophénone-3, 4-méthylbenzylidène camphre, éthylhexyl bis-isopentylbenzoxazolylphenyl mélamine, et leurs combinaisons, dans laquelle le composé de dibenzoylméthane est le 4-tert-butyl-4'-méthoxy dibenzoylméthane.

9. Composition d'écran solaire selon la revendication 1, comprenant en outre un photostabilisant, qui est de préférence choisi dans un groupe constitué de méthoxycrylène, 2,6-naphtalate de diéthylhexyle, syringylidènemalonate de diéthylhexyle, et leurs combinaisons.

10. Composition d'écran solaire selon la revendication 1, comprenant en outre un actif ou agent choisi dans un groupe constitué d'amine de sucre, vitamines, agent de contrôle d'huile, photostérols, composés d'hexamidine, agents tenseurs, principes actifs anti-rides, principes actifs anti-atrophie, flavonoïdes, composés d'acide N-acyl aminé, rétinoïdes, peptides, matériaux particulaires, agents anti-cellulite, principes actifs contre la desquamation, principes actifs anti-acnéiques, antioxydants, agents anti-radicaux libres, agents de conditionnement, agents anti-inflammatoires, principes actifs de bronzage, agent de décoloration de la peau, extraits botaniques, agents actifs antimicrobiens, agents actifs antifongiques, agents actifs antibactériens, agents actifs anti-transpirants, sensates, conservateurs, agents actifs anti-pelliculaires, polymères de substantivité, agents tensioactifs détersifs, et leurs combinaisons.

11. Composite UV améliorant le facteur de protection solaire FPS, pour addition à une composition d'écran solaire, ledit composite UV comprenant un élastomère de silicone gonflé dans un agent actif aux UV liquide de telle sorte que le rapport de l'agent actif aux UV liquide à l'élastomère de silicone va de 1:1 à 8:1, de préférence le composite UV est constitué pratiquement de l'élastomère de silicone gonflé dans l'agent actif aux UV liquide, et plus préférablement l'élastomère de silicone est un élastomère de silicone organiquement modifié.

12. Composite UV selon la revendication 11, dans lequel l'élastomère de silicone est choisi parmi un groupe constitué de polymère réticulé de polyorganosiloxane, polymère réticulé de polyorganosiloxane à modification polyoxyalkylène et polymère réticulé de polyorganosiloxane à modification polyglycérine.

13. Composite UV selon la revendication 11, dans lequel l'élastomère de silicone est choisi parmi un groupe constitué de polymère réticulé alkyldiméthicone/polyglycérine, polymère réticulé diméthicone/polyglycérine, polymère réticulé

diméthicone/poly(propylène glycol), polymère réticulé diméthicone/poly(éthylène glycol), polymère réticulé alkyldiméthicone/poly(propylène glycol), polymère réticulé alkyldiméthicone/poly(éthylène glycol) et polymère réticulé alkyldiméthicone.

**14.** Composite UV selon la revendication 11, dans lequel l'agent actif aux UV liquide est choisi parmi un groupe constitué de 2-éthylhexyl-p-méthoxycinnamate, salicylate d'homométhyle, acide octyldiméthyl-p-aminobenzoïque, 2-éthylhexyl-2-cyano-3,3-diphénylacrylate, 2-éthylhexyl-salicylate, 3,3,5-tri-méthylcclohexylsalicylate, anthranilate de méthyle, et leurs combinaisons, de préférence l'agent actif aux UV liquide est choisi parmi un groupe constitué de salicylate de 2-éthylhexyle, 2-éthylhexyl-p-methoxycinnamate, et leurs combinaisons, et plus préférablement, l'agent actif aux UV liquide comprend un agent actif aux UV solubilisé.

**15.** Composite UV selon la revendication 14, dans lequel l'agent actif aux UV solide est choisi parmi un groupe constitué de composés de dibenzoylméthane, bis-éthylhexyloxyphénol méthoxyphényl triazine, 2,4,6-trianilino-(p-carbo-2'-éthylhexyl-1'-oxy)-1,3,5-triazine, diéthylhexyl butamido triazone, benzoate de diéthylamino hydroxybenzoyl hexyle, benzophénone-3,4-méthylbenzylidène camphre, éthylhexyl bis-isopentylbenzoxazolylphenyl mélamine, et leurs combinaisons, de préférence le composé de dibenzoylméthane est le 4-tert-butyl-4'-méthoxy dibenzoylméthane.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004228821 A
- WO 2007130412 A
- US 5236986 A
- US 5387417 A
- US 5412004 A
- US 5811487 A
- US 20060013791 A1
- US 20060034875 A1
- US 20100183525 A1
- US 20100172849 A1
- US 20100158824 A1
- US 5654389 A
- US 6262170 B
- US 6872401 B
- US 3755560 A
- US 4421769 A
- US 7713519 B
- US 5993789 A
- US 6113931 A
- US 6126925 A
- US 6284916 B
- US 7357919 B
- US 20030108492 A1
- US 20030157035 A
- US 20100183529 A
- US 20080145324 A
- US 200400579912 A
- US 200400579914 A
- US 20000579916 A
- US 2004062726 A
- US 20050220727 A
- US 20080019930 A
- US 20070185038 A1
- US 20060275237 A1
- US 20040175347 A1
- US 20060263309 A1
- US 20070020220 A1
- US 20100112100 A


### Non-patent literature cited in the description


- the Personal Care Product Council's International Cosmetic Ingredient Dictionary and Handbook. 2010
- McCutcheon's, Emulsifiers and Detergents. M. C. Publishing Co, 2010
- the Personal Care Product Council's International Cosmetic Ingredient Dictionary and Handbook. 2006
- Light Stabilizers. the Personal Care Product Council's International Cosmetic Ingredient Dictionary and Handbook. 2010
- The Personal Care Product Council's International Cosmetic Ingredient Dictionary and Handbook. 2010